# EUROPEAN PATENT APPLICATION

(11) **EP 4 406 473 A1**
(43) Date of publication of application: **31.07.2024**
(21) Application number: 22873206.1
(22) Date of filing: 23.09.2022
(51) Int. Cl.: A61B 5/00, A61B 5/346, G16H 50/20

(54) **METHOD, PROGRAM, AND DEVICE FOR DIAGNOSING THYROID DYSFUNCTION ON BASIS OF ELECTROCARDIOGRAM**

(30) Priority: 25.09.2021 KR 20210126785; 20.09.2022 KR 20220118440
(71) Applicant: Medical AI Co., Ltd., Seoul 06302 (KR)
(72) Inventor: KWON, Joonmyoung, Seoul 06302 (KR)
(74) Representative: Patentanwaltskanzlei Matschnig & Forsthuber OG
(86) International application number: PCT/KR2022/014258
(87) International publication number: WO 2023/048502

(57) **Abstract**

According to an embodiment of the present disclosure, there is provided a method of diagnosing thyroid dysfunction based on an electrocardiogram, the method being performed by a computing device including at least one processor, the method including: acquiring electrocardiogram data; and estimating the probability of occurrence of thyroid dysfunction for the subject of measurement of the electrocardiogram data based on the electrocardiogram data by using a pre-trained neural network model; wherein the neural network model is trained based on the correlations between thyroid function and changes in electrocardiogram characteristics.

## Description

### Technical Field

The present disclosure relates to a method of diagnosing thyroid dysfunction, and more particularly to a method of diagnosing thyroid dysfunction based on an electrocardiogram by using a neural network model.

### Background Art

Electrocardiograms (ECGs) are signals used to determine the presence/absence of disease by checking for abnormalities in a conduction system from the heart to electrodes through the measurement of electrical signals generated in the heart.

The heartbeat, which is the cause of the generation of an electrocardiogram, is performed in such a manner that an impulse that originates from the sinus node located in the right atrium first depolarizes the right and left atria, and, after a brief delay in the atrioventricular node, activates the ventricles.

The right ventricle, which has the fastest septum and thin walls, activates before the left ventricle, which has thick walls. The depolarization waves transferred to the Purkinje fibers spread from the endocardium to the epicardium like wavefronts in the myocardium, thus causing ventricular contraction. Electrical impulses are normally conducted through the heart, and thus the heart contracts approximately 60 to 100 times per minute. Each contraction is represented by heart rate per beat.

Such electrocardiograms can be detected through bipolar leads, which record the potential differences between two portions, and unipolar leads, which record the potentials of the portions to which electrodes are attached. Methods of measuring electrocardiograms include standard limb leads, which are bipolar leads, unipolar limb leads, which are unipolar leads, and precordial leads, which are unipolar leads.

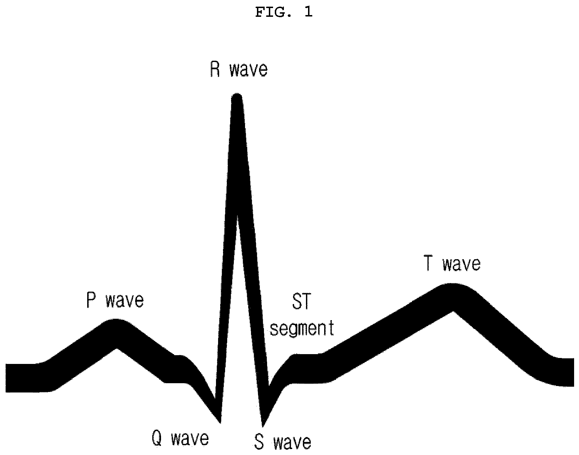

The electrical activity period of the heart is basically divided into atrial depolarization, ventricular depolarization, and ventricular repolarization stages. These individual stages are reflected in the shapes of several waves called P, Q, R, S, and T waves, as shown in FIG. 1.

The electrical activity of the heart can be considered to be normal only when these waves have standard shapes. In order to determine whether these waves have standard shapes, it is necessary to check whether characteristics, such as the times for which the individual waves are maintained, the intervals between the individual waves, the amplitudes of the individual waves, and kurtosis, are within normal ranges.

Such electrocardiogram is measured with an expensive measurement device and used as an auxiliary tool to measure the health condition of a patient. In general, the electrocardiogram measurement device only displays measurement results, and diagnosis is entirely the responsibility of a doctor.

Currently, research is continuing to rapidly and accurately diagnose diseases based on electrocardiograms by using artificial intelligence in order to reduce dependence on doctors. Furthermore, with the development of wearable, lifestyle electrocardiogram measurement devices, there is a rising possibility of diagnosing and monitoring not only heart diseases but also various other diseases based on electrocardiograms.

In particular, in the case of diseases related to thyroid function, early detection is difficult because routine examinations are not performed and also the symptoms thereof are not clear. There is a rising possibility of diagnosing thyroid dysfunction in its early stage by detecting minute changes in electrocardiograms.

### Disclosure

### Technical Problem

The present disclosure has been conceived in response to the above-described background technology, and an object of a method for diagnosing thyroid dysfunction according to an embodiment of the present disclosure is to provide a method for estimating the probability of occurrence of thyroid dysfunction for the subject of the measurement of electrocardiogram data based on the electrocardiogram data by using a neural network model.

However, the objects to be accomplished by the present disclosure are not limited to the object mentioned above, and other objects not mentioned may be clearly understood based on the following description.

### Technical Solution

According to an embodiment of the present disclosure for accomplishing the above-described object, there is provided a method of diagnosing thyroid dysfunction based on an electrocardiogram, the method being performed by a computing device including at least one processor, the method including: acquiring electrocardiogram data; and estimating the probability of occurrence of thyroid dysfunction for the subject of measurement of the electrocardiogram data based on the electrocardiogram data by using a pre-trained neural network model; wherein the neural network model is trained based on the correlations between thyroid function and changes in electrocardiogram characteristics.

Alternatively, there may be provided the method, wherein the neural network model includes a first sub-neural network model that has been trained based on electrocardiogram data measured with 12 multiple leads.

Alternatively, there may be provided the method, wherein the neural network model further includes a second sub-neural network model that has been trained based on at least six limb leads or six precordial leads.

Alternatively, there may be provided the method, wherein the neural network model further includes a third sub-neural network model that has been trained based on electrocardiogram data measured with single leads.

Alternatively, there may be provided the method, wherein the neural network model includes a neural network including a plurality of residual blocks, and the neural network including the residual blocks receives the electrocardiogram data and outputs the probability of occurrence of overt hyperthyroidism.

Alternatively, there may be provided the method, wherein the overt hyperthyroidism corresponds to a case where a free thyroxine level is higher than a predetermined reference range or a case where a thyroid-stimulating hormone level is lower than a reference range.

Alternatively, there may be provided the method, wherein the neural network model includes neural networks corresponding to the plurality of respective leads of the electrocardiogram data and outputs of the neural networks are concatenated into one to derive the probability of occurrence of thyroid dysfunction.

Alternatively, there may be provided the method, wherein the correlations between thyroid function and changes in electrocardiographic characteristics are based on electrocardiographic characteristics, including at least one of the frequency of tachycardia, the length of a QT interval, the bias direction of P, R, and T waves, and QRS duration.

Alternatively, there may be provided the method, wherein the probability of occurrence of thyroid dysfunction increases as the frequency of tachycardia increases.

Alternatively, there may be provided the method, wherein the probability of occurrence of thyroid dysfunction increases as the length of the QT interval increases.

Alternatively, there may be provided the method, wherein the probability of occurrence of thyroid dysfunction increases as the bias direction of P, R, and T waves is directed to the right.

Alternatively, there may be provided the method, wherein the probability of occurrence of thyroid dysfunction increases as the QRS duration becomes shorter.

Alternatively, there may be provided the method, wherein estimating the probability of occurrence of thyroid dysfunction for the subject of measurement of the electrocardiogram data based on the electrocardiogram data by using the pre-trained neural network model includes estimating the probability of occurrence of thyroid dysfunction for the subject of measurement of the electrocardiogram data by inputting biological data including at least one of age and gender, together with the electrocardiogram data, to the neural network model.

According to another embodiment of the present disclosure, there is provided a computer program stored in a computer-readable storage medium, the computer program performing operations for diagnosing thyroid dysfunction based on an electrocardiogram when executed on one or more processors, wherein: the operations include operations of: acquiring electrocardiogram data; and estimating the probability of occurrence of thyroid dysfunction for the subject of measurement of the electrocardiogram data based on the electrocardiogram data by using a pre-trained neural network model; and the neural network model is trained based on the correlations between thyroid function and changes in electrocardiogram characteristics.

According to another embodiment of the present disclosure, there is provided a computing device for diagnosing thyroid dysfunction based on an electrocardiogram, the computing device including: a processor including at least one core; and memory including program codes that are executable on the processor; wherein the processor, in response to the execution of the program codes, acquires electrocardiogram data, and estimates the probability of occurrence of thyroid dysfunction for the subject of measurement of the electrocardiogram data based on the electrocardiogram data by using a pre-trained neural network model; and wherein the neural network model is trained based on the correlations between thyroid function and changes in electrocardiogram characteristics.

### Advantageous Effects

The method for diagnosing thyroid dysfunction according to an embodiment of the present disclosure may provide the method for estimating the probability of occurrence of thyroid dysfunction for the subject of the measurement of electrocardiogram data based on the electrocardiogram data by using the neural network model.

### Description of Drawings

FIG. 1 is a diagram showing an electrocardiogram signal according to the present disclosure;
FIG. 2 is a block diagram of a computing device according to an embodiment of the present disclosure;
FIG. 3 is a flowchart showing a method of diagnosing thyroid dysfunction based on an electrocardiogram according to an embodiment of the present disclosure;
FIG. 4 is a diagram showing the architecture of a neural network model according to an embodiment of the present disclosure;
FIG. 5 is a diagram showing a process for the validation study of a neural network model according to an embodiment of the present disclosure;
FIG. 6 is a diagram showing the performance test results of a neural network model according to an embodiment of the present disclosure; and
FIG. 7 is a diagram showing the electrocardiogram analysis results of subgroups classified by gender and age according to an embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "n-th (n is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

"Data" used herein may include "images," signals, etc. The term "image" used herein may refer to multidimensional data composed of discrete image elements. In other words, "image" may be understood as a term referring to a digital representation of an object that can be seen by the human eye. For example, "image" may refer to multidimensional data composed of elements corresponding to pixels in a two-dimensional image. "Image" may refer to multidimensional data composed of elements corresponding to voxels in a three-dimensional image.

The term "block" used herein may be understood as a set of components classified based on various criteria such as type, function, etc. Accordingly, the components classified as each "block" may be changed in various manners depending on the criteria. For example, a neural network "block" may be understood as a set of neural networks including one or more neural networks. In this case, it can be assumed that the neural networks included in the neural network "block" perform the same specific operations. The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

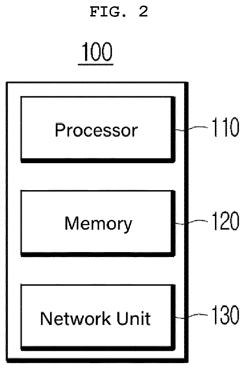

FIG. 2 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 2, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may train a neural network model that diagnoses thyroid dysfunction based on medical data. For example, the processor 110 may train the neural network model to estimate the occurrence and progress of hyperthyroidism based on biological data including information such as gender and age, together with electrocardiogram data. More specifically, the processor 110 may train the neural network model so that the neural network model detects changes in electrocardiograms attributable to the occurrence of hyperthyroidism by inputting electrocardiogram data and various types of biological data to the neural network model. In this case, the neural network model may be trained based on the correlations between thyroid function and changes in electrocardiograms. The correlations between thyroid function and changes in electrocardiograms may be understood as information about the relationships between changes in thyroid function and morphological changes in electrocardiogram signals. The processor 110 may perform an operation representative of at least one neural network block included in the neural network model during the process of training the neural network model.

The processor 110 may estimate whether thyroid dysfunction has occurred based on medical data by using the neural network model generated through the above-described training process. The processor 110 may generate inference data representative of the results of estimation of the probability of occurrence of thyroid dysfunction in a person by inputting electrocardiogram data and biological data including information such as age and gender to the neural network model trained through the above-described process. For example, the processor 110 may predict the occurrence and progress of hyperthyroidism by inputting electrocardiogram data to the trained neural network model. The processor 110 may accurately predict the occurrence of thyroid dysfunction by effectively identifying subtle changes in electrocardiograms that are difficult for humans to interpret through the neural network model for diagnosing thyroid dysfunction.

In addition to the examples described above, the types of medical data and the output of the neural network model may be configured in various manners within a range understandable to those skilled in the art based on the content of the present disclosure.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data acquired from the computation process of the processor 110, etc. through communication with the above-described database, server, or computing device.

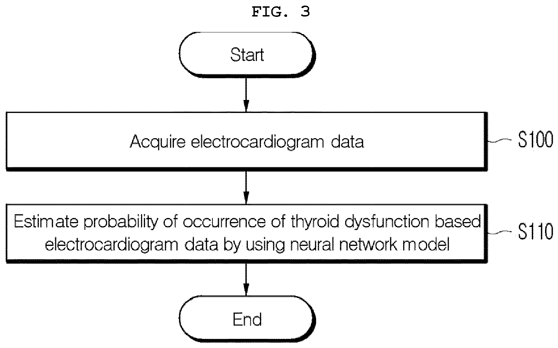

FIG. 3 is a flowchart showing a method of diagnosing thyroid dysfunction based on an electrocardiogram according to an embodiment of the present disclosure.

Referring to FIG. 3, there is shown a method of diagnosing thyroid dysfunction based on an electrocardiogram that is performed by a computing device 100 including at least one processor 110. First, there may be performed step S100 of acquiring electrocardiogram data.

The electrocardiogram data may be acquired directly as the data measured through an electrocardiogram measurement device, or may be acquired from an electrocardiogram measurement device through network communication.

Next, there may be performed step S110 of estimating the probability of occurrence of thyroid dysfunction for the subject of measurement of the electrocardiogram data based on the electrocardiogram data by using a pre-trained neural network model.

Furthermore, the estimation step S110 may include the step of estimating the probability of occurrence of thyroid dysfunction for the measurement subject of the electrocardiogram data by inputting biological data including at least one of age and gender, together with the electrocardiogram data, to the neural network model.

In this case, the neural network model may be trained based on the correlations between thyroid function and changes in electrocardiogram characteristics. Furthermore, the neural network model may be trained based on the correlations between thyroid function and changes in characteristics such as electrocardiograms, gender, and age. More specifically, the neural network model may be trained based on the correlations between the occurrence and progress of hyperthyroidism and changes in electrocardiograms and other characteristics. The neural network model may be used to diagnose various types of thyroid dysfunction such as hypothyroidism as well as hyperthyroidism.

The neural network model may be trained based on electrocardiograms measured with 12 leads and acquired from electrodes of an electrocardiogram measurement device connected to the human body. For example, an electrocardiogram may be measured with 12 leads for ten seconds in length, and may be stored as 500 points per second. Additionally, the neural network model may be trained based on partial information acquired by extracting only 6-limb lead electrocardiograms and single-lead (lead I) electrocardiograms from 12-lead electrocardiograms.

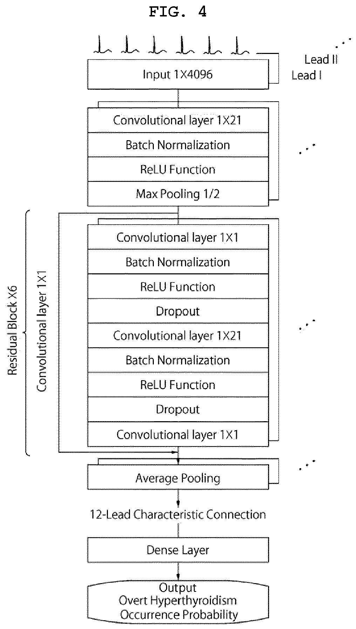

Referring to FIG. 4, there is shown a diagram showing the architecture of a neural network model according to an embodiment of the present disclosure.

Referring to FIG. 4, the neural network model according to the embodiment of the present disclosure may include a neural network composed of a plurality of residual blocks. The neural network composed of the residual blocks may be intended to receive electrocardiogram data and output the probability of occurrence of overt hyperthyroidism.

In this case, overt hyperthyroidism may be diagnosed as having occurred when the free thyroxine level is higher than a predetermined reference range or when the thyroid-stimulating hormone level is lower than a reference range.

More specifically, the neural network model may have the architecture of a ResNet using six residual blocks. Each of the residual blocks may be composed of a convolutional neural network (CNN), batch normalization, a rectified linear unit (ReLU) function, and a dropout layer. The convolutional neural network may be set to one dimension, and the filter size may be set to 21. The input length may be reduced by half whenever three residual blocks out of a total of six residual blocks are passed. A different neural network may be applied to each lead of an electrocardiogram. Average pooling may be applied on a per channel basis at the end of the residual block. The outputs of the neural networks may be concatenated to derive the probability of occurrence of thyroid dysfunction.

The neural network model may include neural networks corresponding to the plurality of leads of electrocardiogram data, respectively. That is, the neural network model may include individual neural networks to which electrocardiograms measured with individual leads are input.

For example, the neural network model may include a first sub-neural network model that has been trained based on electrocardiogram data measured with 12 multiple leads. Furthermore, the neural network model may further include a second sub-neural network model that has been trained based on at least six limb leads or six precordial leads. Furthermore, the neural network model may further include a third sub-neural network model that has been trained based on electrocardiogram data measured with a single lead. The neural network model may selectively use at least one of the first, second, and third sub-neural network models depending on the number of leads. Accordingly, the neural network model may effectively predict the occurrence of thyroid dysfunction regardless of the number of leads. Furthermore, when 12-lead electrocardiogram data is input, the neural network model may output the probability of occurrence of thyroid dysfunction by combining the outputs of the respective sub-models using all of the first, second, and third sub-neural network models. The neural network model may increase the accuracy of prediction of the occurrence of thyroid dysfunction through this combination.

A statistical analysis method performed to validate a neural network model having the above-described architecture will be described below. To check basic features, continuous variables were presented as mean values and standard deviations. Validation results were compared using the unpaired student's t-test or Mann-Whitney u-test. Categorical variables were represented by percentages, and the chi-square test (χ² test) was used.

The performance of the neural network model was validated by comparing the probability calculated by the model with the presence/absence of hyperthyroidism in internal and external validation datasets. The validation was performed with reference to the area under the receiver operating characteristic curve (AUC). Cutoff points were identified using Youden's J statistic in training datasets. Sensitivity, specificity, a positive predictive value, and a negative predictive value were calculated from the internal and external validation datasets by applying the cutoff points. The 95% confidence interval of the AUC was calculated using Sun's and Su's optimization of the Delong method.

A validation study of a neural network model according to an embodiment of the present disclosure will be described below.

To prove the robustness of the neural network model, subgroups were generated according to age and gender and sensitivity analysis was performed. Gender was classified as male and female, and age was classified as the age under 40 years, the age from 40 to 50 years, the age from 50 to 60 years, the age from 60 to 70 years, and the age of 70 years or older.

The hypothesis was formulated that subtle changes might occur in an electrocardiogram during the period prior to hyperthyroidism and the neural network model could detect these small changes and predict the occurrence of the disease. To validate this hypothesis, subgroup analysis was conducted. The external validation dataset was extracted from patients who were diagnosed as normal in their first thyroid function tests (TFTs) and underwent follow-up thyroid function tests. The time interval between the first thyroid function tests and the follow-up thyroid function tests is four weeks or longer. Based on the probabilities of occurrence of hyperthyroidism estimated by the neural network model, the study subjects were classified into a high-risk group and a low-risk group. The cutoff points were determined in the training datasets by using Youden's J statistic. The Kaplan-Meier method was used to analyze the results over 36 months.

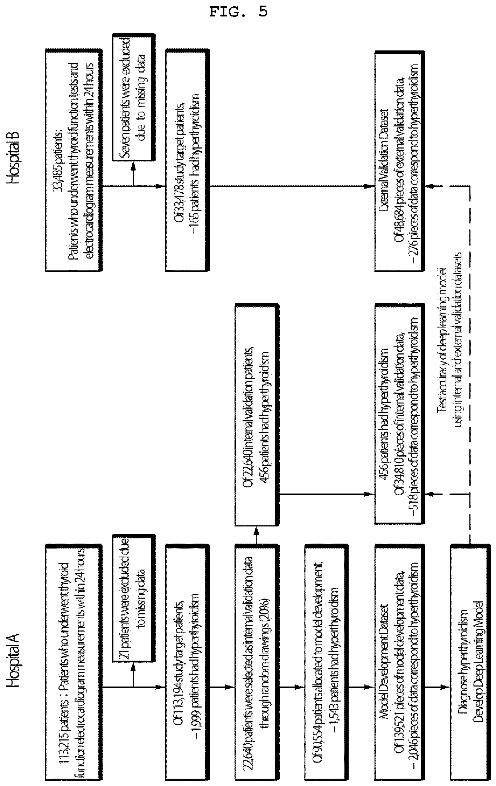

FIG. 5 is a diagram showing a process for the validation study of a neural network model according to an embodiment of the present disclosure.

Referring to FIG. 5, the subjects of the validation study of the neural network model according to the embodiment of the present disclosure were 113,215 patients at hospital A and 33,485 patients at hospital B. Twenty one patients at hospital A and seven patients at hospital B having missing clinical information or electrocardiogram data were excluded. A total of 2,164 patients having hyperthyroidism were included. For neural network training, 139,521 pieces of electrocardiogram data measured from 90,554 patients at hospital A were used. The internal validation used 34,810 pieces of electrocardiogram data measured from 518 patients at hospital A. The external validation used 48,684 pieces of electrocardiogram data measured from 33,478 patients at hospital B. The basic characteristics of a neural network model training cohort (hospital a, n = 113,175) and an external validation cohort (hospital b, n = 33,478) are shown in Table 1 below:

**Table 1**

| | Internal Validation Dataset (hospital a) n=113,194 | | | External Validation Dataset (hospital b) n=33,478 | | | p‡ |
|---|---|---|---|---|---|---|---|
| Characteristics | Non-hyperthyroidism | Hyperthyroidism | p† | Non-hyperthyroidism | Hyperthyroidism | p† | p‡ |
| Study Subject, Number of Persons (%) | 111,195 (98.2) | 1999 (1.8) | | 33,313 (99.5) | 165 (0.5) | | <0.001 |
| Age, Years, Mean (sd) | 43.95 (13.41) | 40.64 (14.38) | <0.001 | 55.37 (15.48) | 51.51 (14.93) | 0.001 | <0.001 |
| Male, Number of Persons (%) | 56808 (51.1) | 638 (31.9) | <0.001 | 15960 (47.9) | 56 (33.9) | <0.001 | <0.001 |
| Heart Rate, bpn (%) | 66.47 (12.75) | 89.37 (19.61) | <0.001 | 70.89 (16.20) | 97.59 (24.19) | <0.001 | <0.001 |
| PR Interval, ms, Mean (sd) | 158.46 (23.63) | 145.83 (25.51) | <0.001 | 166.97 (26.47) | 151.08 (27.45) | <0.001 | <0.001 |
| QRS Length, ms, Mean (sd) | 93.07 (12.00) | 86.24 (11.37) | <0.001 | 94.36 (15.02) | 89.67 (13.46) | <0.001 | <0.001 |
| QTC, ms, Mean (sd) | 418.46 (23.42) | 427.41 (32.29) | <0.001 | 433.44 (31.63) | 442.88 (31.23) | <0.001 | <0.001 |
| P-wave Axis, Mean (sd) | 47.38 (23.68) | 50.37 (24.46) | <0.001 | 44.34 (27.65) | 48.93 (31.43) | 0.045 | <0.001 |
| R-wave Axis, Mean (sd) | 49.42 (32.13) | 54.86 (28.28) | <0.001 | 40.51 (39.55) | 46.27 (33.08) | 0.062 | 0.002 |
| T-wave Axis, Mean (sd) | 39.83 (23.53) | 46.32 (25.33) | <0.001 | 39.38 (38.21) | 47.95 (47.98) | 0.004 | <0.001 |

The alternative hypothesis for p marked with † in Table 1 is that there is a difference between hyperthyroidism and overt hyperthyroidism. The alternative hypothesis for p marked with ‡ is that there is a difference between hospital A (a model development and internal validation data group) and hospital B (an external validation group) for each variable. Gender, age, and incidence for hyperthyroidism statistically differed among hospitals. Patients having hyperthyroidism had more tachycardia and a longer QT interval. Patients having hyperthyroidism exhibited the rightward bias of P-, R-, and T-wave axes and had shorter QRS duration.

In summary, the above-described correlations between the thyroid function and the changes in electrocardiographic characteristics may be based on electrocardiographic characteristics including at least one of the frequency of tachycardia, the length of a QT interval, the bias direction of P, R, and T waves, and QRS duration.

The probability of occurrence of thyroid dysfunction estimated by the neural network model may increase as the frequency of tachycardia increases. The probability of occurrence of thyroid dysfunction may increase as the length of the QT interval increases.

The probability of occurrence of thyroid dysfunction estimated by the neural network model may increase as the P-wave, R-wave, and T-wave bias directions are directed toward the right.

The probability of occurrence of thyroid dysfunction estimated by the neural network model may increase as the QRS duration becomes shorter.

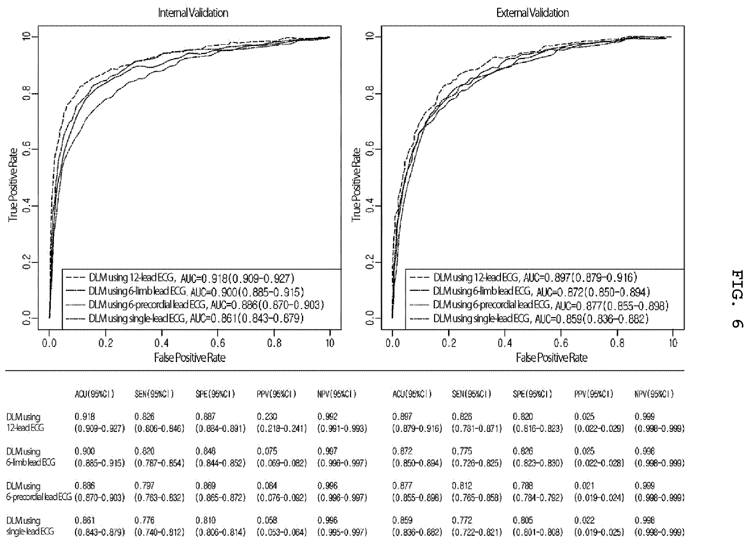

FIG. 6 is a diagram showing the performance test results of a neural network model according to an embodiment of the present disclosure.

Referring to FIG. 6, in the internal and external validation, AUC denotes the area under the receiver operating characteristic curve of the neural network model, DLM denotes the neural network model, electrocardiogram denotes the electrocardiogram, NPV denotes the negative predictive value, PPV denotes the positive predictive value, SEN denotes sensitivity, and SPE denotes specificity.

In the internal and external validation, the AUCs of the neural network models using 12-lead electrocardiograms were 0.918 (0.909-0.927) and 0.897 (0.879-0.916), respectively. Through sensitivity analysis, the robustness of the neural network model based on gender and age was verified. Those identified by the neural network model as high-risk patients showed a significant change in the occurrence of hyperthyroidism compared to those identified as low-risk patients (p < 0.01). The performances of the neural network models using 6-lead electrocardiograms or single-lead electrocardiograms were verified. Referring to Table 2, in the sensitivity analysis for gender and age, the performances of all the models had an AUC value of 0.830 or higher.

**Table 2**

| | Male | | | | | Female | | | | |
|---|---|---|---|---|---|---|---|---|---|---|
| Age | AUC | SEN | SPE | PPV | NPV | AUC | SEN | SPE | PPV | NPV |
| -39 | 0.919 (0.899 -0.940) | 0.839 (0.793 -0.884) | 0.883 (0.877 -0.890) | 0.169 (0.148 -0.190) | 0.995 (0.993 -0.996) | 0.932 (0.913 -0.951) | 0.841 (0.794 -0.889) | 0.897 (0.891 -0.903) | 0.173 (0.151 -0.196) | 0.995 (0.994 -0.997) |
| 40-49 | 0.893 (0.868 -0.917) | 0.790 (0.736 -0.844) | 0.872 (0.865 -0.879) | 0.131 (0.113 -0.149) | 0.994 (0.992 -0.996) | 0.911 (0.887 -0.934) | 0.809 (0.759 -0.859) | 0.915 (0.910 -0.920) | 0.166 (0.144 -0.187) | 0.996 (0.994 -0.997) |
| 50-59 | 0.880 (0.853 - 0.907) | 0.823 (0.768 - 0.877) | 0.803 (0.795 - 0.810) | 0.072 (0.061 - 0.083) | 0.996 (0.994 - 0.997) | 0.904 (0.877 - 0.931) | 0.812 (0.755 - 0.870) | 0.855 (0.848 - 0.862) | 0.099 (0.083 0.114) | 0.996 (0.994 - 0.997) |
| 60-69 | 0.830 (0.787 - 0.874) | 0.682 (0.595 - 0.769) | 0.876 (0.868 - 0.883) | 0.079 (0.061 - 0.096) | 0.994 (0.993 - 0.996) | 0.868 (0.829 - 0.908) | 0.754 (0.675 - 0.833) | 0.840 (0.831 - 0.850) | 0.084 (0.067 -0.101) | 0.994 (0.992 -0.996) |
| 70- | 0.873 (0.827 -0.919) | 0.836 (0.751 -0.921) | 0.825 (0.814 -0.835) | 0.064 (0.049 -0.080) | 0.997 (0.996 -0.999) | 0.852 (0.804 -0.900) | 0.957 (0.900 -1.015) | 0.674 (0.663 -0.685) | 0.019 (0.014 -0.025) | 1.000 (0.999 -1.000) |

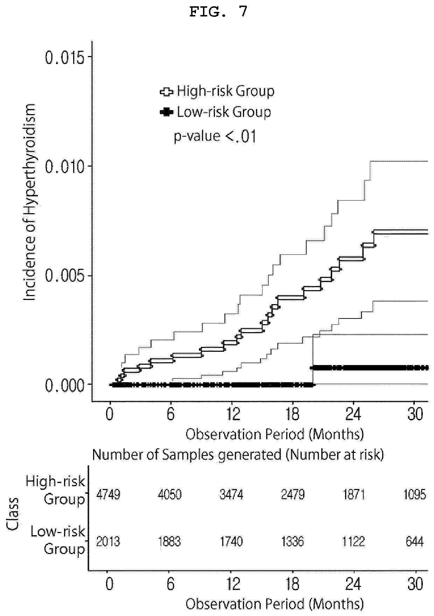

FIG. 7 is a diagram showing the electrocardiogram analysis results of subgroups classified by gender and age according to an embodiment of the present disclosure. Referring to FIG. 7, the subgroup analysis was conducted using the follow-up thyroid function test results of 6,762 patients who were diagnosed as normal in first thyroid function tests. Among these, 24 patients developed hyperthyroidism. The subjects of the subgroup analysis were divided into a high-risk group of 4,749 people and a low-risk group of 2,013 people according to the probabilities of occurrence of hyperthyroidism output by the neural network model. It was found that the high-risk group had a significantly higher risk of the occurrence of hyperthyroidism than the low-risk group (0.48% vs. 0.05%, p < 0.01).

Meanwhile, thyroid function is closely related to the cardiovascular system, and may affect the cardiovascular system as well as heart function, vascular resistance, and autonomic cardiovascular control. In particular, thyroid hormone-mediated changes may affect the improvement of cardiac function through enhanced ventricular contraction (inotropy) and heart rate (chronotropy). Signs and symptoms of thyroid dysfunction may be determined to be the results of thyroid hormones affecting the heart and the cardiovascular system. Thyroid dysfunction may be associated with increased cardiovascular disease incidence and mortality. Moreover, when hyperthyroidism was not treated, the risk of cardiovascular disease was higher than that when the disease was treated. In both a case where hyperthyroidism was treated and a case where hyperthyroidism was not treated, the incidence of cardiovascular disease increased in accordance with the duration in which the thyroid-stimulating hormone level decreased.

Therefore, for the early detection and prediction of thyroid dysfunction having such a risk, the method of diagnosing thyroid dysfunction according to an embodiment of the present disclosure may estimate the probability of occurrence of thyroid dysfunction for the subject of the measurement of electrocardiogram data based on the electrocardiogram data by using the neural network model.

Furthermore, the method for diagnosing thyroid dysfunction according to an embodiment of the present disclosure has the effect of diagnosing hyperthyroidism using the neural network model based on information such as electrocardiograms, gender, and age.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of diagnosing thyroid dysfunction based on an electrocardiogram, the method being performed by a computing device including at least one processor, the method comprising:
acquiring electrocardiogram data; and
estimating a probability of occurrence of thyroid dysfunction for a subject of measurement of the electrocardiogram data based on the electrocardiogram data by using a pre-trained neural network model;
wherein the neural network model is trained based on correlations between thyroid function and changes in electrocardiogram characteristics.

2. The method of claim 1, wherein the neural network model includes a first sub-neural network model that has been trained based on electrocardiogram data measured with 12 multiple leads.

3. The method of claim 1, wherein the neural network model includes a second sub-neural network model that has been trained based on at least six limb leads or six precordial leads.

4. The method of claim 1, wherein the neural network model includes a third sub-neural network model that has been trained based on electrocardiogram data measured with single leads.

5. The method of claim 1, wherein:
the neural network model includes a neural network including a plurality of residual blocks; and
the neural network including the residual blocks receives the electrocardiogram data and outputs a probability of occurrence of overt hyperthyroidism.

6. The method of claim 5, wherein the overt hyperthyroidism corresponds to a case where a free thyroxine level is higher than a predetermined reference range or a case where a thyroid-stimulating hormone level is lower than a reference range.

7. The method of claim 1, wherein:
the neural network model includes neural networks corresponding to a plurality of respective leads of the electrocardiogram data; and
outputs of the neural networks are concatenated into one to derive the probability of occurrence of thyroid dysfunction.

8. The method of claim 1, wherein the correlations between thyroid function and changes in electrocardiographic characteristics are based on electrocardiographic characteristics, including at least one of a frequency of tachycardia, a length of a QT interval, a bias direction of P, R, and T waves, and QRS duration.

9. The method of claim 8, wherein the probability of occurrence of thyroid dysfunction increases as the frequency of tachycardia increases.

10. The method of claim 8, wherein the probability of occurrence of thyroid dysfunction increases as the length of the QT interval increases.

11. The method of claim 8, wherein the probability of occurrence of thyroid dysfunction increases as the bias direction of P, R, and T waves is directed to a right.

12. The method of claim 8, wherein the probability of occurrence of thyroid dysfunction increases as the QRS duration becomes shorter.

13. The method of claim 1, wherein estimating the probability of occurrence of thyroid dysfunction for the subject of measurement of the electrocardiogram data based on the electrocardiogram data by using the pre-trained neural network model comprises:
estimating the probability of occurrence of thyroid dysfunction for the subject of measurement of the electrocardiogram data by inputting biological data including at least one of age and gender, together with the electrocardiogram data, to the neural network model.

14. A computer program stored in a computer-readable storage medium, the computer program performing operations for diagnosing thyroid dysfunction based on an electrocardiogram when executed on one or more processors, wherein:
the operations include operations of:
acquiring electrocardiogram data; and
estimating a probability of occurrence of thyroid dysfunction for a subject of measurement of the electrocardiogram data based on the electrocardiogram data by using a pre-trained neural network model; and
the neural network model is trained based on correlations between thyroid function and changes in electrocardiogram characteristics.

15. A computing device for diagnosing thyroid dysfunction based on an electrocardiogram, the computing device comprising:
a processor including at least one core; and
memory including program codes that are executable on the processor;
wherein the processor, in response to execution of the program codes, acquires electrocardiogram data, and estimates a probability of occurrence of thyroid dysfunction for a subject of measurement of the electrocardiogram data based on the electrocardiogram data by using a pre-trained neural network model; and
wherein the neural network model is trained based on correlations between thyroid function and changes in electrocardiogram characteristics.
